# EUROPÄISCHE PATENTSCHRIFT

(11) **EP 1 744 797 B1**
(45) Veröffentlichungstag und Bekanntmachung des Hinweises auf die Patenterteilung: **16.07.2008**
(21) Anmeldenummer: 05736301.2
(22) Anmeldetag: 10.05.2005
(51) Int. Cl.: A61M 1/10

(54) **VORRICHTUNG ZUR EPIKARDIALEN UNTERSTÜTZUNG UND/ODER ÜBERNAHME DER HERZTÄTIGKEIT**
DEVICE FOR THE EPICARDIAL SUPPORT AND/OR RESUMPTION OF CARDIAC ACTIVITY
DISPOSITIF D'ASSISTANCE EPICARDIQUE ET/OU DE REPRISE DE L'ACTIVITE CARDIAQUE

(30) Priorität: 11.05.2004 DE 102004023192
(43) Veröffentlichungstag der Anmeldung: 24.01.2007
(73) Patentinhaber: PPA Technologies AG, 07745 Jena (DE)
(72) Erfinder: FERRARI, Markus, 07747 Jena (DE)
(74) Vertreter: Weidener, Jörg Michael
(86) Internationale Anmeldenummer: PCT/EP2005/005052
(87) Internationale Veröffentlichungsnummer: WO 2005/110513

(56) Entgegenhaltungen:
- US-A- 3 034 501
- US-A- 3 513 836
- US-A- 5 169 381
- US-A- 5 738 627

## Beschreibung

Die vorliegende Erfindung betrifft eine Vorrichtung zur epikardialen Unterstützung und/oder Übernahme der Herztätigkeit, mit einer Doppelmembran, die eine elastische innere Membran und eine nicht dehnbare äußere Membran sowie einen dazwischen gebildeten, geschlossenen und mittels eines Fluids inflatierbaren und deflatierbaren Hohlraum mit einer dem rechten Ventrikel zugeordneten ersten Kammer und einer dem linken Ventrikel zugeordneten zweiten Kammer sowie eine die beiden Kammern separierende Trennwand aufweist.

Eine Vorrichtung zur epikardialen Unterstützung und/oder Übernahme der Herztätigkeit der vorgenannten Art ist bereits aus der US-A-3 034 501 bekannt. Bei der bekannten Vorrichtung wird jede Kammer separat mit Fluid versorgt, was eine entsprechende Anzahl an Fluidschläuchen und Ventilen bedeutet und zu einer relativ aufwendigen Bedienung der gesamten Vorrichtung führt.

Des weiteren ist aus der DE 199 51 220 A1 ein perkutan implantierbares System zur mechanischen Unterstützung und zum temporären Ersatz der Pumpfunktion des Herzens bekannt. Die bekannte Vorrichtung wird nach Sondierung des Herzbeutels in zusammengefaltetem Zustand perkutan in den Herzbeutel eingebracht oder am Ende einer Operation in dem Herzbeutel chirurgisch positioniert und dort mit der Doppelmembran um die rechte und linke Herzkammer gelegt. Dabei ist die Vorrichtung in deflatiertem Zustand so dünn, daß eine Kompression der Nachbarorgane vermieden wird. Nach der Implantation wird der Hohlraum der Doppelmembran über einen Verbindungsschlauch rhythmisch mit einem Fluid beaufschlagt, welches entweder ein Gas (Helium oder CO₂) oder eine geeignete Flüssigkeit sein kann.

Durch dieses rhythmische Inflatieren oder Deflatieren des Hohlraums der Doppelmembran und weil die äußere Membran im Gegensatz zur inneren Membran nicht dehnbar ist, kommt es zu einer Druckübertragung und Kompression des Herzens über die das Herz umschließende Doppelmembran. Dabei wird das Blut aus der rechten Herzkammer in die Pulmonalarterie und gleichzeitig aus der linken Kammer in die Aorta ausgetrieben oder bei vorhandener Pumpfunktion des Herzens wird die systolische Auswurfarbeit des Herzmuskels unterstützt.

Aus der US-A-5 169 381 ist eine Vorrichtung zur Unterstützung der Herztätigkeit mit einer eine innere und eine äußere Membran sowie einen dazwischen gebildeten, geschlossenen und mittels eines Fluids inflatierbaren und deflatierbaren Hohlraum aufweisenden Doppelmembran bekannt. Diese Doppelmembran weist einen in axialer Richtung verlaufenden keilförmigen Schlitz auf, der sich über ¾ der vertikalen Ausdehnung der Doppelmembran erstreckt, und ist darüber hinaus mit einer dem rechten Ventrikel zugeordneten ersten Kammer und einer dem linken Ventrikel zugeordneten zweiten Kammer versehen. Jede dieser beiden Kammern wird ebenfalls separat mit Fluid versorgt, was zu den eingangs genannten Nachteilen führt.

Aufgabe der vorliegenden Erfindung ist es, eine Vorrichtung zu Unterstützung und/oder Übernahme der Herztätigkeit der eingangs genannten Art zur Verfügung zu stellen, deren Bedienbarkeit vereinfacht ist.

Diese Aufgabe ist bei einer Vorrichtung der eingangs genannten Art erfindungsgemäß dadurch gelöst, daß die erste Kammer und die zweite Kammer über wenigstens ein in der Trennwand angeordnetes Ventil miteinander in Verbindung stehen.

Die Vorteile der erfindungsgemäßen Lösung liegen insbesondere darin, daß nur ein Fluidschlauch zum Inflatieren und Deflatieren des Hohlraums der Doppelmembran erforderlich ist und dennoch eine Augmentation nur eines Ventrikels durch regionale Inflation bzw. Deflation möglich ist. Die so geschaffene Vorrichtung ist einfach zu bedienen, und es können je nach Bedarf Doppelmembranen hergestellt werden, welche die alleinige Augmentation des rechten Ventrikels oder - in einer anderen Ausführungsform - nur des linken Ventrikels, beides unter Beibehaltung der Möglichkeit eine Unterstützung beider Ventrikel gleichzeitig, ermöglichen.

Durch die erfindungsgemäße Art der Kammerung und mechanische Modifikation der Doppelmembran durch wenigstens ein internes Ventil ist also eine isolierte Augmentation einer einzigen Herzkammer möglich, wobei die Doppelmembran die andere Herzkammer zur mechanischen Stabilisierung umschließt und so zur Stabilisierung im Sinne eines Widerlages beiträgt. Somit kann bei isoliertem Rechtsherzversagen eine vornehmliche Augmentation des rechten Ventrikels erfolgen, während die Doppelmembran den linken Ventrikel nur passiv umschlingt. Ein Öffnen oder Schließen des Ventils ermöglicht ein Umschalten zwischen den so gegebenen zwei Betriebsmodi, nämlich "Gesamt-Augmentation" bzw. "Regionale Augmentation".

Vorteilhafte Weiterbildungen der Erfindung sind in den Unteransprüchen 2 bis 4 angegeben.

Vorzugsweise ist das Ventil, welches die beiden Kammern miteinander verbindet und in der die beiden Kammern separierenden Trennwand angeordnet ist, von außen auf- bzw. zusteuerbar. Das bedeutet, dass das Ventil beispielsweise mittels eines IR-Signals oder mittels eines Funksignals bedienbar ist. Die Vorteile dieser Weiterbildung liegen darin, dass keine zusätzliche Steuerleitung für das Ventil erforderlich ist und somit ein weiteres Risiko für den Patienten vermieden wird.

Da nach koronaren Bypass-Operationen eine externe Kompression der epikardialen Gefäße nicht wünschenswert ist, weist die Doppelmembran im Bereich der großen Herzkranzgefäße vorzugsweise variable Aussparungen auf. Eine solche Doppelmembran kann entweder individuell auf den Patienten angepasst angefertigt oder aber, wie gemäß einer weiteren vorteilhaften Weiterbildung vorgesehen, mittels verschiebbarer Abstützungen an die besonderen Erfordernisse eines Patientenherzens angepasst werden. Dabei können die variablen Aussparungen durch faltbare, flexible Stege oder Halbschläuche durch mechanische Manipulation des Chirurgen in die gewünschte Position gebracht werden. Diese faltbaren, flexiblen Stege oder Halbschläuche können während des Pumpbetriebes entweder durch selbsthaftende Eigenschaften, Anwendung eines Gewebe-Klebstoffes, durch eine Halteschiene, oder durch Rillen innerhalb der Doppelmembran, welches bestimmte Positionen vorgeben, in der gewünschten Position gehalten werden.

Im folgenden wird ein Ausführungsbeispiel der Erfindung anhand einer Zeichnung näher erläutert.

Es zeigen:
- Figur 1:: eine schematische Darstellung der erfindungsgemäßen Vorrichtung mit Augmentation nur des rechten Ventrikels in der Systole;
- Figur 2:: eine der Figur 1 vergleichbare Darstellung in der Diastole; und
- Figur 3:: die Darstellung eines menschlichen Herzens mit aufgesetzter Vorrichtung und Aussparungen.

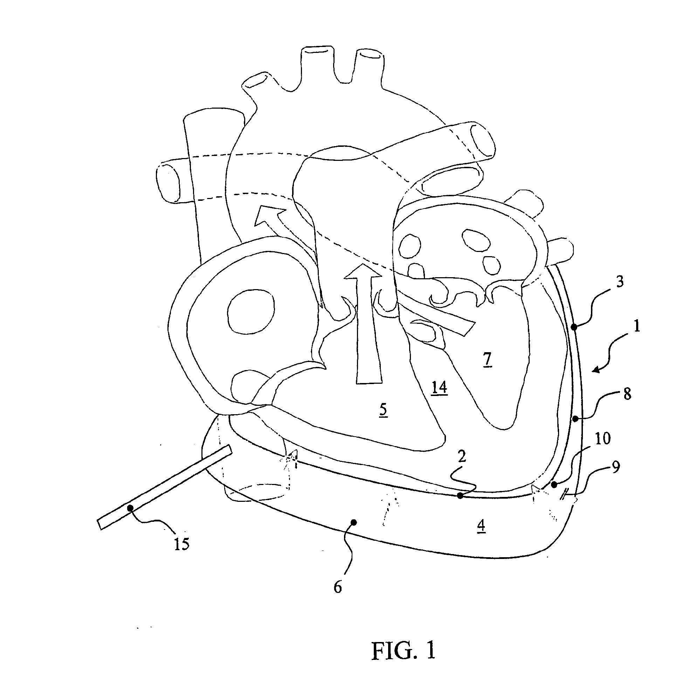

Figur 1 zeigt eine schematische Darstellung einer Vorrichtung zur epikardialen Unterstützung und/oder Übernahme der Herztätigkeit, mit einer Doppelmembran 1, die eine elastische innere Membran 2 und eine nicht dehnbare äußere Membran 3 sowie einen dazwischen gebildeten, geschlossenen und über einen Fluidschlauch 15 mittels eines Fluids inflatierbaren und deflatierbaren Hohlraum 4 aufweist. Der Hohlraum 4 ist mittels einer Trennwand 10 in eine dem rechten Ventrikel 5 zugeordnete erste Kammer 6 und eine dem linken Ventrikel 7 zugeordnete zweite Kammer 8 aufgeteilt. Beide Kammern 6, 8 stehen über wenigstens ein in der Trennwand 10 angeordnetes Ventil 9 miteinander in Verbindung. Dabei ist das Ventil 9 bzw. alle in jener Trennwand angeordneten Ventile (es ist nur eines dargestellt) von außen beispielsweise mittels eines IR- oder Funk-Signals auf- bzw. zusteuerbar.

Die nach innen auf das Herz 14 weisenden drei Pfeile innerhalb der inflatierten Kammer 6 des rechten Ventrikels 5 deuten an, dass die Darstellung eine Momentaufnahme der Systole mit Augmentation nur des rechten Ventrikels 5 durch eine regionale Inflation der Doppelmembran 1 wiedergibt.

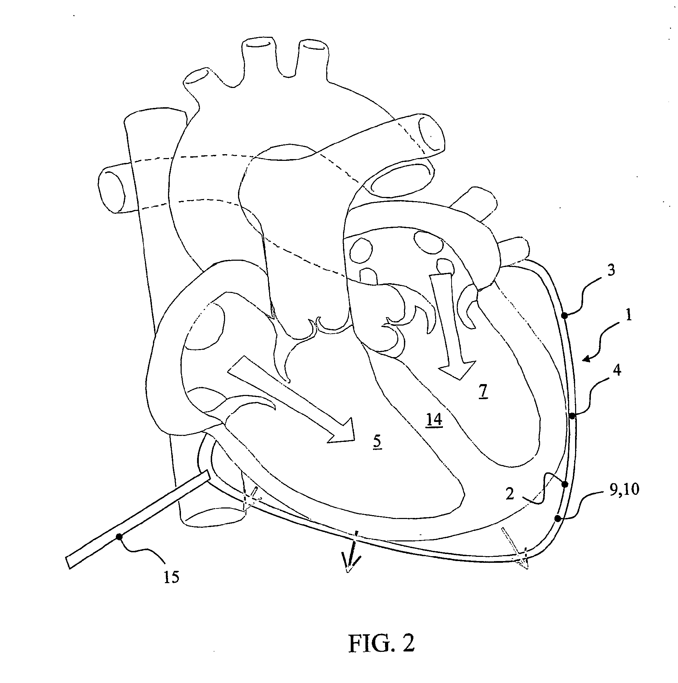

Figur 2 zeigt eine der Figur 1 vergleichbare Darstellung in der Diastole. Dabei wird die Füllung des rechten Ventrikels 5 durch Deflation der rechten Kammer 6 der Doppelmembran 1 augmentiert. Das Ventil 9 der Trennwand 10 ist in diesen Fällen, wo eine Augmentation nur des rechten Ventrikels 5 gewünscht ist, geschlossen gehalten. Nur dann, wenn eine Augmentation beider Ventrikel 5, 7 gewünscht ist, werden sowohl die erste Kammer 6 als auch die zweite Kammer 8 inflatiert bzw. deflatiert.

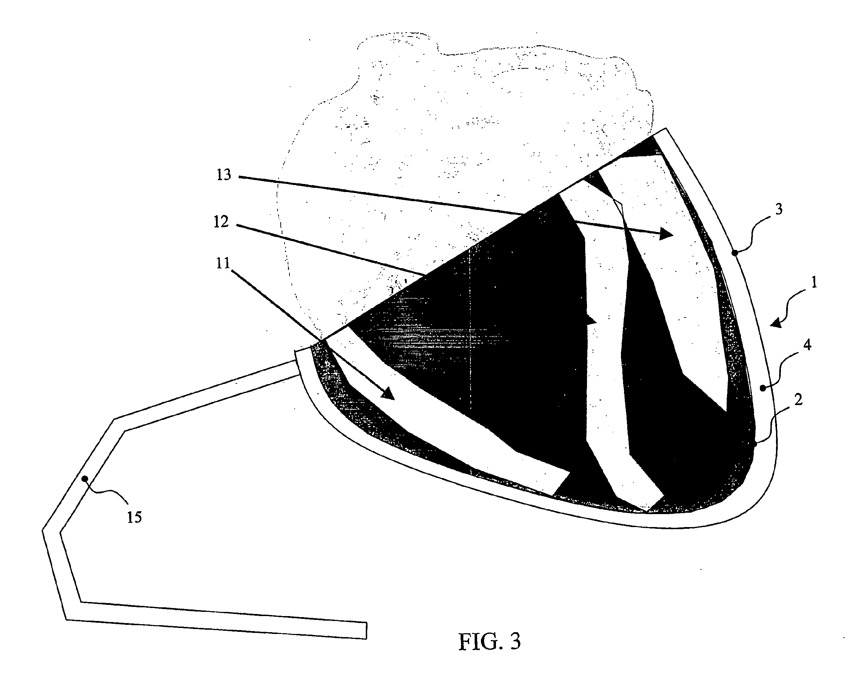

Figur 3 zeigt ein Patientenherz mit einer schematisch darumgelegten Doppelmembran 1, deren Hohlraum 4 wiederum über den Fluidschlauch 22 inflatierbar und deflatierbier ist. Dieses Ausführungsbeispiel der Doppelmembran 1 weist im Bereich der großen Herzkranzgefäße variable Aussparungen 11, 12, 13 auf, um eine externe Kompression der epikardialen Gefäße zu vermeiden. Diese Aussparungen 11, 12, 13 sind durch verschiebbare Abstützungen, die hier nicht dargestellt sind, an die Erfordernisse eines Patientenherzens anpassbar.

## Patentansprüche

1. Vorrichtung zur epikardialen Unterstützung und/oder Übernahme der Herztätigkeit, mit einer Doppelmembran (1), die eine elastische innere Membran (2) und eine nicht dehnbare äußere Membran (3) sowie einen dazwischen gebildeten, geschlossenen und mittels eines Fluids inflatierbaren und deflatierbaren Hohlraum (4) mit einer dem rechten Ventrikel (7) zugeordneten ersten Kammer (6) und einer dem linken Ventrikel (7) zugeordneten zweiten Kammer (8) sowie eine die beiden Kammern (6, 8) separierende Trennwand (10) aufweist,
**dadurch gekennzeichnet, daß**
die erste Kammer (6) und die zweite Kammer (8) über wenigstens ein in der Trennwand (10) angeordnetes Ventil (9) miteinander in Verbindung stehen.

2. Vorrichtung nach Anspruch 1,
**dadurch gekennzeichnet, daß**
das Ventil (9) von außen auf- bzw. zusteuerbar ist.

3. Vorrichtung nach Anspruch 1 oder 2,
**gekennzeichnet durch**
Aussparungen (11, 12, 13) in der Doppelmembran (1) im Bereich der großen Herzkranzgefäße.

4. Vorrichtung nach Anspruch 3,
**gekennzeichnet durch**
verschiebbare Abstützungen, mittels derer die Aussparungen (11, 12, 13) an die Erfordernisse eines Patientenherzens anpaßbar sind.

## Claims

1. Device for epicardial support and/or the assuming of cardiac activity having a double membrane (1) consisting of an elastic inner membrane (2) and a non-expandable outer membrane (3) as well as a closed cavity (4) formed therebetween which can be inflated and deflated by means of a fluid exhibiting a first chamber (6) allocated to the right ventricle (5) and a second chamber (8) allocated to the left ventricle (7) as well as a dividing wall (10) separating the two chambers (6, 8),
**characterized in that**
the first chamber (6) and the second chamber (8) are connected to one another by means of at least one valve (9) arranged in the dividing wall (10).

2. Device according to claim 1,
**characterized in that**
the valve (9) is externally opened and/or closed in a controlled manner.

3. Device according to claim 1 or 2,
**characterized by**
recesses (11, 12, 13) in the double membrane (1) in the area of the large coronary artery.

4. Device according to claim 3,
**characterized by**
displaceable supports by means of which the recesses (11, 12, 13) can be adapted to the requirements of a patient's heart.

## Revendications

1. Dispositif pour le soutien épicardique et/ou pour assumer l'activité cardiaque, avec une double membrane (1), qui présente une membrane (2) interne élastique et une membrane (3) externe non extensible ainsi qu'un espace creux (4) formé entre celles-ci, fermé et pouvant être gonflé et dégonflé au moyen d'un fluide avec une première chambre (6) associée au ventricule droit (7) et une deuxième chambre (8) associée au ventricule gauche (7), ainsi qu'une paroi de séparation (10) séparant les deux chambres (6, 8), **caractérisé en ce que** la première chambre (6) et la deuxième chambre (8) sont reliées via au moins une soupape (9) disposée dans la paroi de séparation (10).

2. Dispositif selon la revendication 1, **caractérisé en ce que** la soupape (9) peut être commandée ou ajustée à partir de l'extérieur.

3. Dispositif selon la revendication 1 ou 2, **caractérisé par** des évidements (11, 12, 13) dans la double membrane (1) au niveau des grands vaisseaux coronaires.

4. Dispositif selon la revendication 3, **caractérisé par** des appuis pouvant être déplacés au moyen desquels les évidements (11, 12, 13) peuvent être adaptés aux exigences d'un coeur d'un patient.
